(19) Europäisches Patentamt
European Patent Office
Office européen des brevets

(11) EP 2 433 948 A1

(12) EUROPEAN PATENT APPLICATION

(43) Date of publication:
**28.03.2012 Bulletin 2012/13**

(51) Int Cl.:
***C07H 19/10*** *(2006.01)* ***A61K 31/7072*** *(2006.01)*
***A61P 31/04*** *(2006.01)*

(21) Application number: **10075522.2**

(22) Date of filing: **25.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Max-Planck-Gesellschaft zur Förderung der Wissenschaften e.V.**
**80539 München (DE)**

(72) Inventors:
- **Meinhart, Anton**
**68165 Mannheim (DE)**
- **The other inventor has agreed to waive his entitlement to designation.**

(74) Representative: **Arth, Hans-Lothar**
**ABK Patent Attorneys**
**Jasminweg 9**
**14052 Berlin (DE)**

# (54) Use of a new class of nucleotide sugar as antibiotic or cytostatic agent

(57) The present invention relates to the use of a compound of the formula (A), its use in medicine and for the prophylaxis and/or treatment of infectious diseases. Also disclosed are pharmaceutical formulations containing at least one of the inventive compounds. The compounds are especially useful for prophylaxis and/or treatment of bacterial infection. Further part of the invention is a method for producing the compounds of the formula (A).

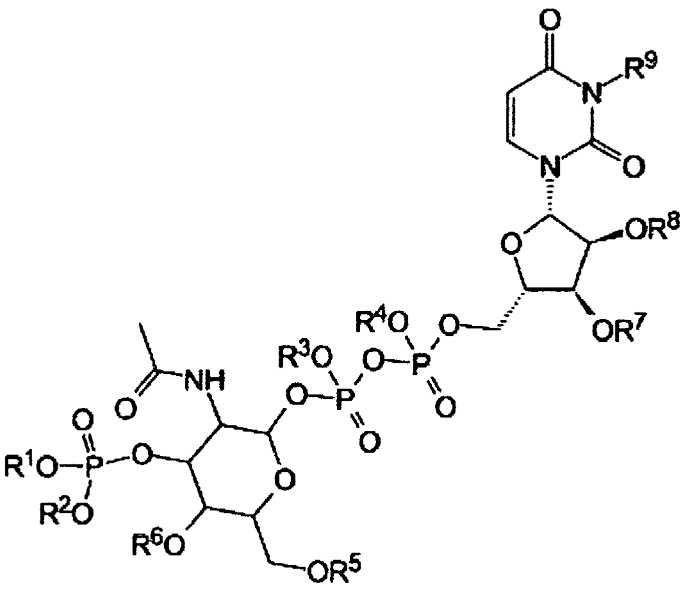

**(A)**

EP 2 433 948 A1

## Figure 2

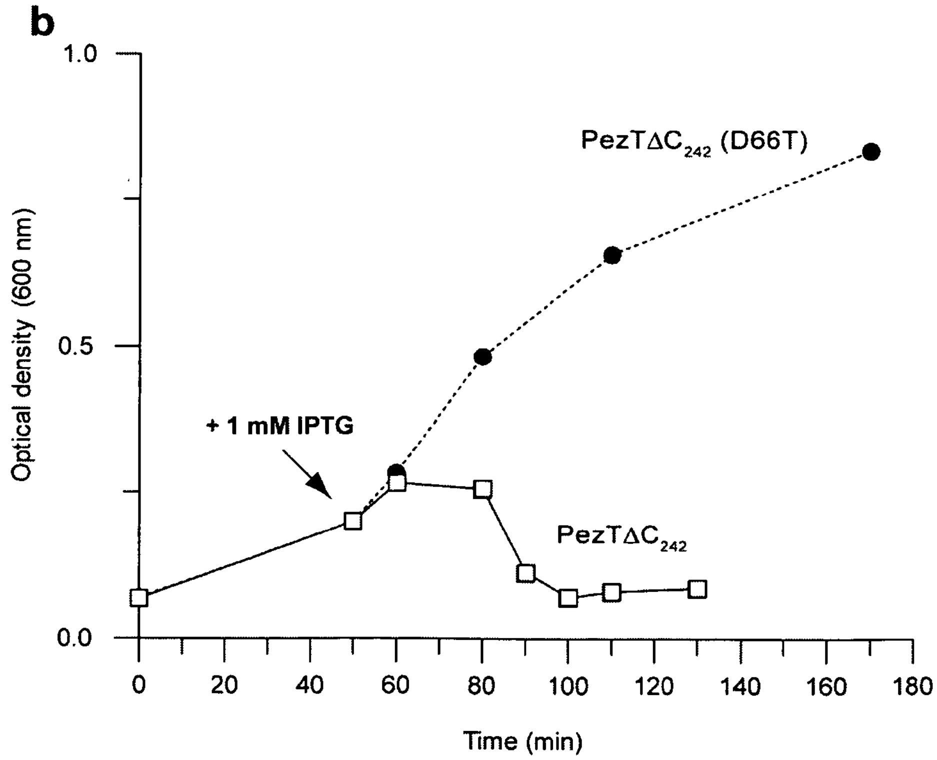
b
1.0
0.5
0.0
Optical density (600 nm)
PezTΔC242 (D66T)
+ 1 mM IPTG
PezTΔC242
0
20
40
60
80
100
120
140
160
180
Time (min)

SEQUENCE LISTING

<110> Max-Planck-Gesellschaft zur Förderung der Wissenschaften e.V.

<120> Use of a new class of nucleotide sugar as antibiotic or cytostatic agent

<130> GAR-P03199EP

<160> 5

<170> PatentIn version 3.3

<210> 1
<211> 253
<212> PRT
<213> Streptococcus pneumoniae

<400> 1

```
Met Glu Ile Gln Asp Tyr Thr Asp Ser Glu Phe Lys His Ala Leu Ala
1               5                   10                  15

Arg Asn Leu Arg Ser Leu Thr Arg Gly Lys Lys Ser Ser Lys Gln Pro
            20                  25                  30

Ile Ala Ile Leu Leu Gly Gly Gln Ser Gly Ala Gly Lys Thr Thr Ile
        35                  40                  45

His Arg Ile Lys Gln Lys Glu Phe Gln Gly Asn Ile Val Ile Ile Asp
    50                  55                  60

Gly Asp Ser Phe Arg Ser Gln His Pro His Tyr Leu Glu Leu Gln Gln
65                  70                  75                  80
```

```
Glu Tyr Gly Lys Asp Ser Val Glu Tyr Thr Lys Asp Phe Ala Gly Lys
                85                  90                  95

Met Val Glu Ser Leu Val Thr Lys Leu Ser Ser Leu Arg Tyr Asn Leu
            100                 105                 110

Leu Ile Glu Gly Thr Leu Arg Thr Val Asp Val Pro Lys Lys Thr Ala
        115                 120                 125

Gln Leu Leu Lys Asn Lys Gly Tyr Glu Val Gln Leu Ala Leu Ile Ala
    130                 135                 140

Thr Lys Pro Glu Leu Ser Tyr Leu Ser Thr Leu Ile Arg Tyr Glu Glu
145                 150                 155                 160

Leu Tyr Ile Ile Asn Pro Asn Gln Ala Arg Ala Thr Pro Lys Glu His
                165                 170                 175

His Asp Phe Ile Val Asn His Leu Val Asp Asn Thr Arg Lys Leu Glu
            180                 185                 190

Glu Leu Ala Ile Phe Glu Arg Ile Gln Ile Tyr Gln Arg Asp Arg Ser
        195                 200                 205

Cys Val Tyr Asp Ser Lys Glu Asn Thr Thr Ser Ala Ala Asp Val Leu
    210                 215                 220

Gln Glu Leu Leu Phe Gly Glu Trp Ser Gln Val Glu Lys Glu Met Leu
225                 230                 235                 240

Gln Val Gly Glu Lys Arg Leu Asn Glu Leu Leu Glu Lys
                245                 250
```

<210> 2
<211> 15
<212> DNA
<213> Artificial

<220>
<223> Forward Primer

<400> 2
gccgcactcg agcac 15

<210> 3
<211> 40
<212> DNA
<213> Artificial

<220>
<223> Reverse Primer

<400> 3
agatttctcg agcgctgctg ccacctgcaa catctccttc 40

<210> 4
<211> 28
<212> DNA
<213> Artificial

<220>
<223> Forward Primer

<400> 4
atcatagatg gtgatagttt tcgttctc 28

<210> 5

<211> 28

<212> DNA

<213> Artificial

<220>

<223> Reverse Primer

<400> 5

gagaacgaaa actatcacca tctatgat 28

## Description

The present invention relates to the use of a compound of the formula (A), its use in medicine and for the prophylaxis and/or treatment of infectious diseases. Also disclosed are pharmaceutical formulations containing at least one of the inventive compounds. The compounds are especially useful for prophylaxis and/or treatment of bacterial infection. Further part of the invention is a method for producing the compounds of the formula (A)

Toxin-antitoxin (TA) systems were initially discovered on low copy number plasmids where they function as post-segregational killing systems that help ensure the segregational stability of plasmids. TA systems usually consist of two genes: the toxin gene encodes a stable protein whereas the antitoxin gene encodes either a labile protein or an untranslated, antisense RNA species. The toxic effect is neutralized by inhibition of toxin translation when the antitoxin is an RNA (type I), or by strong binding of the cognate antitoxin when the antitoxin is a protein (type II). When encoded on plasmids, TA systems were known as "addiction modules," because cells which lose these plasmids would be killed, thus causing the cells to be "addicted" to the short-lived antitoxin product because its *de novo* synthesis is essential for cell survival.

All TA-systems, except the epsilon/zeta TA-systems, known to date, target macromolecules involved in translation or replication. Epsilon/zeta systems, where the toxic function remained unknown, are widespread among plasmids with homologues found in human pathogens including *Streptococcus pyogenes* and vancomycin-resistant strains of *Enterococcus faecium* and *Staphylococcus aureus.* Additionally, chromosomally encoded epsilon/zeta systems (PezA/PezT for pneumococcal epsilon/zeta antitoxin toxin system) have also been recently identified on different streptococcal integrative and conjugative elements.

The present inventors investigated the enzymatic activity of zeta toxins and characterized their substrates and products. Additionally they could show that the enzymatic activity of zeta toxins is indeed the toxic function in vivo. In fact, they were able to purify the product from cells that have been poisoned by the toxin and showed that the product of zeta toxins inhibits peptidoglycan synthesis by inhibiting the enzyme MurA.

MurA (UDP-N-acetylglucosamine enolpyruvyl transferase, EC 2.5.1.7) catalyzes the first committed step in the synthesis of the bacterial cell wall. It is the target of the naturally occurring, broad-spectrum antibiotic fosfomycin. Fosfomycin, an epoxide, is a relatively poor drug because an ever-increasing number of bacteria have developed resistance to fosfomycin. Thus, there is a critical need for the development of novel drugs that target MurA by a different molecular mode of action. The present inventors have surprisingly identified a new scaffold of potent MurA inhibitors, namely derivatives of Uridinediphosphate-2-N-acetyl-3-phosphate-glucosamie. They are competitive inhibitors of MurA with respect to the substrate, UDP-N-acetylglucosamine (UNAG).

Therefore the present invention relates to compounds having the general formula (A):

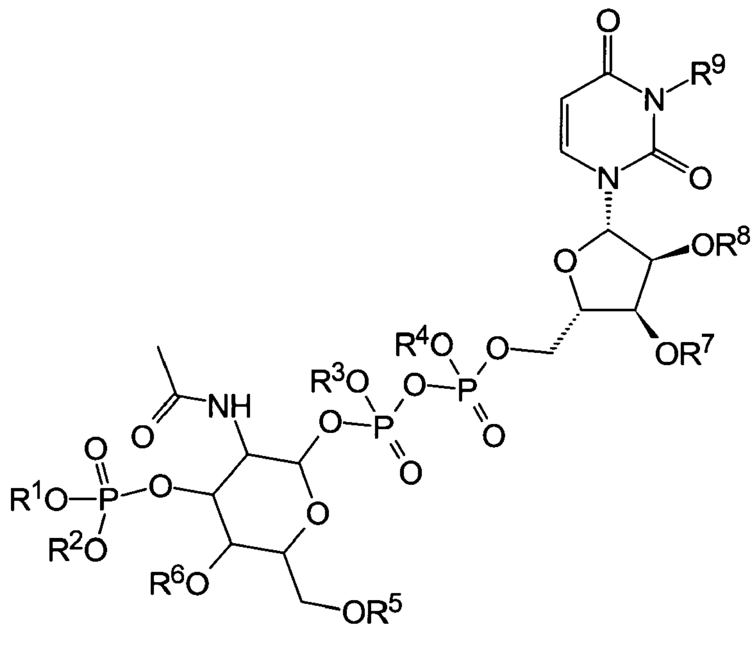

**(A)**

wherein
$R^1$. $R^2$, $R^3$, $R^4$ and $R^9$ represent independently of each other
-H, $-CH_3$, $-C_2H_5$, $-C_3H_7$, $-CH(CH_3)_2$, $-C(CH_3)_3$ $-C_4H_9$, $-C_5H_{11}$, $-C_6H_{13}$, cyclo-$C_3H_5$, cyclo-$C_5H_7$, cydo-$C_5H_9$, cyclo-$C_6H_{11}$, $-CH_2O-COCH_3$, $-CH_2O-COC_2H_5$, $-CH_2O-COC_3H_7$ $-CH_2O-COC_4H_9$, $-CH_2O-COC_5H_{11}$, $-CH_2O-COC_6H_{13}$, $-CH_2O-COC(CH_3)_3$, $-CH_2O-COOCH_3$, $-CH_2O-COOC_2H_5$, $-CH_2O-COOC_3H_7$- $CH_2O-COOC_4H_9$, $-CH_2O-COOC_5H_{11}$, $-CH_2O-COOC_6H_{13}$, $-CH_2O-COOC(CH_3)_3$ and $-CH(R^a)-C_6H_4-COOR^b$
wherein $-C_6H_4-$ is a phenylen moiety which is either substituted ortho, meta or para and $R^a$ represents -H or $-CH_2-COOR^b$ and $R^b$ represents independently of $R^a$ -H, $-CH_3$, $-C_2H_5$, $-C_3H_7$, $-CH_2(CH_3)$, $-CH(CH_3)_2$, $-C(CH_3)_3$, $-C_4H_9$, $-C_5H_{11}$ or $-C_6H_{13}$;
or
instead of the substituents disclosed above, $R^1$ and $R^2$ can form together one of the following residues:

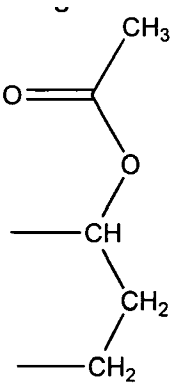 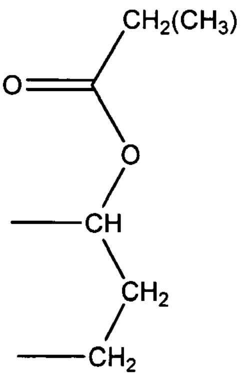 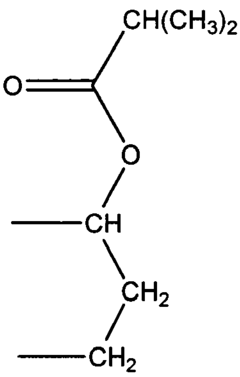 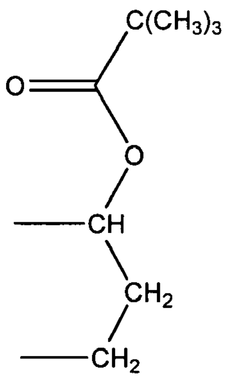

$R^5$, $R^6$, $R^7$, $R^8$ and $R^9$ represent independently of each other
-H, -CHO, $-COCH_3$, $-COC_2H_5$, $-COC_3H_7$, $-COCH(CH_3)_2$, $-COC_4H_9$, $-COC_5H_{11}$, $-COC_6H_{13}$, -CO-cyclo-$C_3H_5$, -CO-cyclo-$C_4H_7$, -CO-cyclo-$C_5H_9$, -CO-cyclo-$C_6H_{11}$;
or
instead of the substituents disclosed above, $R^5$ and $R^6$ can form together one of the following residues:

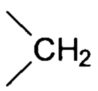 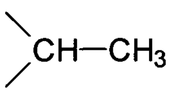 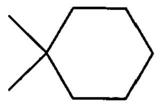 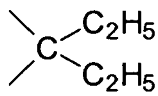

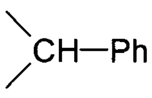 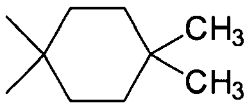 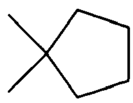 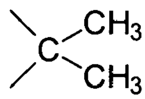

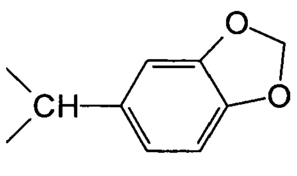 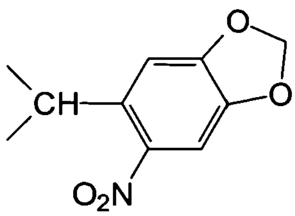 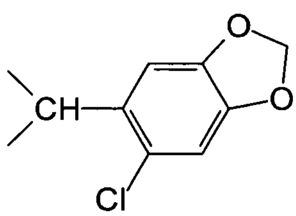

instead of the substituents disclosed above, $R^7$ and $R^8$ can form together one of the following residues:

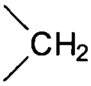 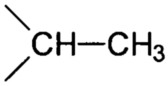 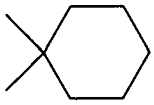 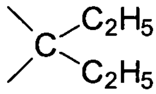

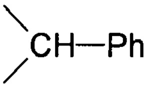 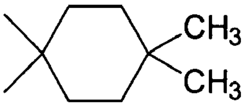 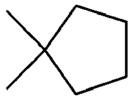 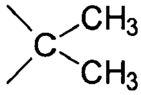

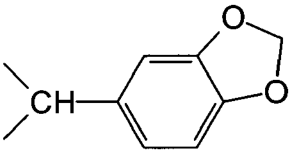 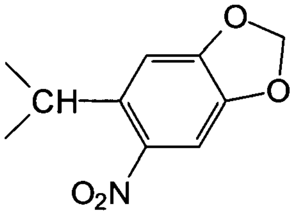 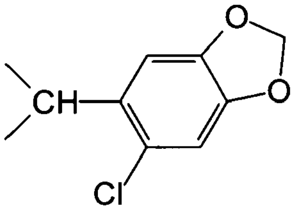

Preferably, the compounds of the present invention have the general formula (B)

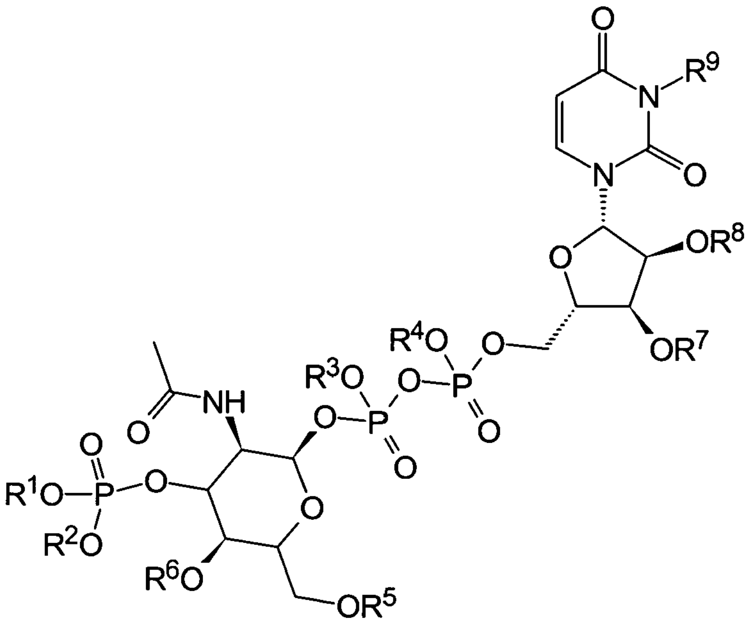

**(B)**

wherein the substituents $R^1$ to $R^9$ have the meaning as disclosed for general formula (A).

Also preferred are compounds having the general formula (C):

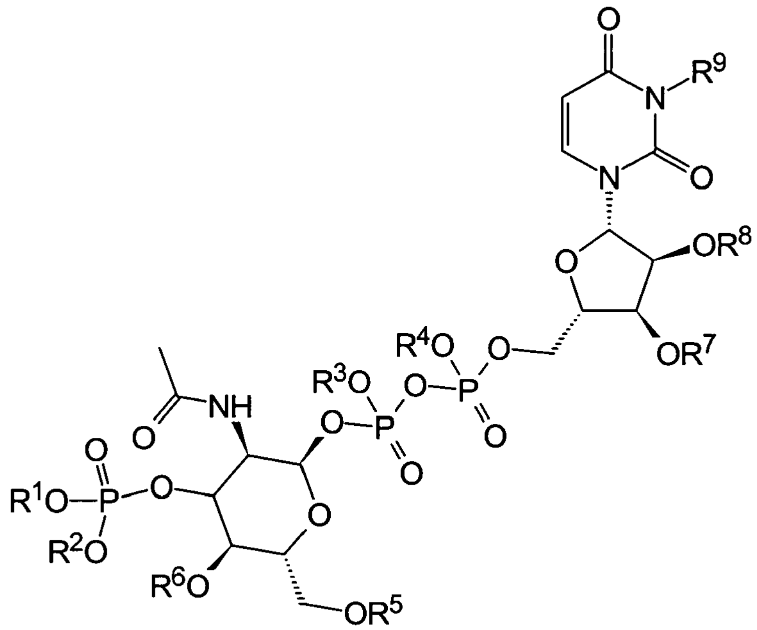

**(C)**

wherein the substituents $R^1$ to $R^9$ have the meaning as disclosed for general formula (A).

One preferred compound of the present invention is Uridinediphosphate-2-N-acetyl-3-phosphate-glucosamine (UNAG-3P). The present invention also comprises pharmaceutically acceptable salts of the compounds according to the general formula (A) and all stereoisomeric forms of the compounds according to the general formula (A).

Another aspect of the present invention relates to the use of the inventive compounds as drugs, i.e. as pharmaceutically active agents applicable in medicine.

Surprisingly it was found that the above-mentioned compounds of the general formula (A) as well as the pharmaceutical compositions comprising said compounds are useful for treatment of infectious diseases.

Thus, the nucleotide sugars of the present invention can be used for prophylaxis and treatment of infectious diseases preferably bacterial infections, or for the preparation of a pharmaceutical formulation for the prophylaxis and treatment of infectious diseases and preferably bacterial infections. Preferably, the bacterial infection is caused by a bacteria of a genus selected from Allochromatium, Acinetobacter, Bacillus, Campylobacter, Chlamydia, Chlamydophila, Clostridium, Citrobacter, Escherichia, Enterobacter, Enterococcus, Francisella, Haemophilus, Helicobacter, Klebsiella, Listeria, Moraxella, Mycobacterium, Neisseria, Proteus, Pseudomonas, Salmonella, Serratia, Shigella, Stenotrophomonas, Staphyloccocus, Streptococcus, Synechococcus, Vibrio, and Yersina. More preferably, the bacterial infection is selected from *Allochromatium vinosum, Acinetobacter baumanii, Bacillus anthracis, Campylobacter jejuni, Clostridium* spp., *Citrobacter* spp., *Escherichia coli, Enterobacter* spp., *Enterococcus faecalis., Enterococcus faecium, Francisella tularensis, Haemophilus influenzae, Helicobacter pylori, Klebsiella* spp., *Listeria monocytogenes, Moraxella catharralis, Mycobacterium tuberculosis, Neisseria meningitidis, Neisseria gonorrhoeae, Proteus mirabilis, Proteus vulgaris, Pseudomonas aeruginosa, Salmonella* spp., *Serratia* spp., *Shigella* spp., *Stenotrophomonas maltophilia, Staphyloccocus aureus, Staphyloccocus epidermidis, Streptococcus pneunmoniae, Streptococcus pyogenes, Streptococcus agalactiae, Yersina pestis,* and *Yersina enterocolitica,* and the like.

Preferably, the treated bacterial infection is caused by a bacterium that expresses a peptidoglycan biosynthesis pathway, and in particular, expresses the enzyme encoded by the MurAlMurZ gene. Numerous studies have demonstrated that the MurA gene and its paralog MurZ are conserved across a range of Gram positive and Gram negative bacteria; see, for example, Wenseng Du et al., J Bacteriol, 2000.

The present inventors could show that the inventive compounds impair muramic acid synthesis in different ways. The synthesis of muramic acids is regulated by a negative feedback loop in which MurA is inhibited by its downstream product UDP-N-acetylmuramic acid in E. coli. The inventive compounds of the general formula (A) are competitive inhibitors of MurA; and additionally hijack the feedback regulatory loop. The inventive compounds will interfere with the peptidoglycan synthesis in Gram-negative and Gram-positive organisms, since MurA is conserved in most prokaryotes (H. Barreteau et al., FEMS Microbiol. Rev (2008)). The inventive nucleotide sugars will also interfere with lipid A synthesis

of Gram-negative bacteria, since condensation of the lipid anchor and UNAG is performed via the aminosugar 3' hydroxyl group of UNAG. Thus, the inventive compounds of the general formula (A) ubiquitously interfere with synthesis of a variety of cell wall components, independent of the cell wall architecture. The compounds of the general formula (A) can also be administered in form of their pharmaceutically active salts optionally using substantially nontoxic pharmaceutically acceptable carrier, excipients, adjuvants or diluents. The medications of the present invention are prepared in a conventional solid or liquid carrier or diluents and a conventional pharmaceutically-made adjuvant at suitable dosage level in a known way. The preferred preparations and formulations are in administratable form which is suitable for oral application. These administratable forms, for example, include pills, tablets, film tablets, coated tablets, capsules, powders and deposits. Other than oral administratable forms are also possible. The inventive compounds of the formula (A) or pharmaceutical preparations or formulations containing said compounds may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

Within the disclosed methods the pharmaceutical compositions of the present invention, containing at least one nucleotide sugar compound of the general formula (A) or pharmaceutically acceptable salts thereof as an active ingredient will typically be administered in admixture with suitable carrier material suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.

Also included are solid form preparations which are intended to be converted, shortly before use, to liquid form preparations for either oral or parenteral administration. Such liquid forms include solutions, suspensions and emulsions.

The inventive nucleotide sugars of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to active ingredients dispersed or solubilized in a hydrophilic semi-solid matrix. Powders for

constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'i-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1% to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the nucleotide sugars of the present invention may be found in "Remington's Pharmaceutical Sciences" Mack Publishing Co., Easton PA. A suitable composition comprising at least one compound of the invention and/or pharmaceutically acceptable salts thereof may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of a compound of the general formula (A) refers to that amount of the compound that results in an at least partial inhibition of bacterial cell wall synthesis. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. The actual amount of the composition administered will be dependent on the subject being treated, on the subject's weight, the severity of the affliction, the manner of administration and the judgement of the prescribing physician.

The present invention further refers to a method for the production of compounds of the general formula (A) comprising the steps:

a) Providing a recombinant protein having a sequence of SEQ ID NO:1 or a homolog thereof having the same catalytic activity and
b) Adding ATP and nucleotide sugars
c) Purifying the compounds from the reaction mixture

Optionally, step d) follows after step c) of the inventive method.

d) Chemically modifying the compound of step c)

Preferably, the method of the present invention uses a recombinant protein having a sequence of SEQ ID NO: 1 or allelic variants and homologs thereof having at least 70-80%, preferably at least 80-90%, more preferably at least 90-95%, and most preferably at least 95-99.9% or more identity with the sequences of SEQ ID NO:1.

It is preferred if the nucleotide sugars of step b) of the inventive method are selected from UDP-N-acetylglucosamine or UDP-N-acetylgalactosamine.

The compounds produced by the inventive method may be purified by anion exchange chromatography subsequently desalted and concentrated in a Speed Vac Concentrator as shown by the example 1.

**Description of the figures**

<u>**Fig. 1**. Enzymatic activity of PezTΔ$C_{242}$ S. *pneumoniae* and zeta from S. *pyogenes.*</u>

(a) Samples containing 0.25 mM UNAG, 5 mM $MgCl_2$, 1 mM ATP and 1 μM PezTΔ$C_{242}$ (solid line) or additionally with 1 μM PezA antitoxin (dashed line) were analyzed after 1 h of incubation at 25°C by anion exchange chromatography. (b) Analysis of the equivalent reaction using 1 μM epsilon/zeta complex from S. *pyogenes* after 2 h (dashed line) and 24 h (solid line) of incubation. Note that UNAG turnover was slower but in contrast to the PezAT system, zeta is active despite the presence of stoichiometric amounts of the epsilon antitoxin. This is most probably caused by the different toxin-antitoxin affinities in the two TA-systems. (c) Schematic reaction mechanism of UNAG-3P formation by zeta toxins.

<u>**Fig. 2.** Formation of UNAG-3P is required for cell death caused by PezT</u>

(a) *In vivo* metabolite extracts from cells after 1 h of PezTΔ$C_{242}$ (middle panel) or inactive PezTΔ$C_{242}$ (D66T) (bottom panel) expression were analyzed by high pressure liquid chromatograpy using a strong anion exchange column. The chromatogram of authentic standards is shown in the top panel. Note that individual concentrations of isolated small molecules cannot be compared quantitatively, since concentrations of individual runs were adjusted to their total absorbance at 260 nm. (b) The enzymatic formation of UNAG-3P by PezT is required for cell death and only expression of active PezTΔ$C_{242}$ leads to lysis (□). In contrast, cells expressing enzymatically inactive PezTΔ$C_{242}$ (D66T) show no lytic phenotype and continue to grow (●).

<u>**Fig. 3.** UNAG-3P inhibits the essential enzyme MurA</u>

(a) MurA performs the first step of UDP-muramic acid biosynthesis. After sequential binding of UNAG and phosphoenolpyruvate, a tetrahedral intermediate is formed that yields enolpyruvyl-UNAG after cleavage of inorganic phosphate. UNAG-3P most likely mimics this tetrahedral intermediate and thereby inhibits MurA catalysis by a competitive binding mechanism. (b) Determination of the $K_i$ of UNAG-3P for MurA. Enolpyruvyl-UNAG formation catalyzed by MurA from *E. coli* in presence of PEP and UNAG-3P and varying UNAG concentrations under steady state conditions. MurA enzyme kinetics were followed by coupling the reaction to phosphate dependent cleavage of fluorescent 7-methylguanosine by nucleoside phosphorylase resulting in a strong decrease of the fluorescence at 400 nm ($\lambda_{exc}$ = 300 nm). The UNAG-3P concentrations for each saturation curve were 0 μM (●), 15 μM (□) and 30 μM (Δ). A Lineweaver-Burk plot is shown as inset. The saturation curves were fitted globally by nonlinear regression assuming competitive inhibition (—) yielding a $K_m$ of 15 μM for UNAG and a $K_i$ of 7 μM for UNAG-3P.

**Fig. 4.** UNAG-3P can be produced on a preparative scale.

(a) Preparative MonoQ run after phosphorylation of UNAG by PezTΔ$C_{242}$. The reaction was supplied with an excess of UNAG over ATP to ensure nearly complete turnover of ATP to ADP allowing baseline separation of UNAG-3P from residual ATP. After a wash with 0.2 M ammonium acetate (pH 8.0) releasing excess UNAG, the three nucleotide species were eluted with a gradient from 0.2 M to 1 M ammonium acetate. The absorbance at 260 nm is shown in black and the absorbance at 280 nm is shown in grey. Note that the optical cell was saturated due to the high absorbance. Since ammonium acetate is a volatile salt, the majority of it evaporates in a subsequent vacuum concentration step. (b) The isolated UNAG-3P can be separated conveniently from residual salt and other impurities using a gelfiltration column such as a Superdex 75.

## Examples

**Example 1:** Cloning, expression and purification of PezT plasmids and proteins.

E. *coli* DH5α was used as a host strain for all cloning experiments. E. *coli* BL21-CodonPlus (DE3)-RIL (Stratagene) was used for protein overexpression.

The plasmid containing the non-toxic PezTΔ$C_{242}$ (D66T) open-reading-frame with C-terminal $His_6$-tag (pET28b (*pezT*Δ$C_{242}$*(D66T)*)) was derived from the pET28b(pezT(D66T)) clone described previously (S. K. Khoo et al., J. Biol. Chem. (2007)) by PCR amplification with 5'-GCCGCACTCGAGCAC-3' as forward and 5'-AGATTTCTCGAGCGCTGCTGCCACCTGCAACATCTCCTTC-3'as reverse primer using the QuikChange kit (Stratagene). The amplified vector DNA was linearized with XhoI and methylated template DNA was removed by a DpnI nuclease digest. The resulting vector DNA lacking a coding sequence for the last nine amino acids was recircularized using the Rapid DNA Ligation Kit (Fermentas), yielding pET28b(pezTΔ$C_{242}$(D66T)). Wild type coding sequence at the active site and thus toxicity of the construct was restored by site-directed mutagenesis of the codon triplet for Thr66 to Asp66 using the QuikChange kit with 5'-ATCATAGATGGTGATAGTTTTCGTTCTC-3' as forward and 5'-GAGAACGAAAACTATCACCATCTATGAT-3' as reverse primer, yielding pET28b(pezTΔ$C_{242}$). Shock expression of PezTΔ$C_{242}$ was induced by addition of IPTG to 1 mM at an $OD_{600}$ = 1.0 for 1.5 h.

*Purification of Recombinant pET28b(pezT*Δ$C_{242}$*)*

After shock expression, poisoned cells were harvested by centrifugation in a SLC-6000 rotor for 20 min at 6000 rpm and 4°C. The cell pellet was resuspended in 30 mL of 50 mM Tris-HCl pH 8.0, 150 mM NaCl and 50 mM ammonium sulfate. Cells were opened by sonication on ice and the supernatant was cleared by 1 h centrifugation in a SS34 rotor at 13'000 rpm (4°C). The cleared lysate was loaded on a 1 mL His-Trap column (GE healthcare), equilibrated with 50 mM Tris-HCl pH 8.0, 150 mM NaCl, 50 mM ammonium sulfate and 20 mM imidazole (buffer A) and washed with 20 column volumes (cv) using the same buffer. After a high-salt wash (20 cv) with 50 mM Tris-HCl pH 8.0 and 1 M NaCl, the column was re-equilibrated with buffer A (10 cv). Subsequently, bound PezTΔ$C_{242}$ was eluted with 50 mM Tris-HCl pH 8.0, 250 mM NaCl, 50 mM ammonium sulfate and 500 mM imidazole. Fractions (10 x 1 mL) were collected and analysed by SDS-electrophoresis.

Pooled fractions were dialyzed over night against 50 mM Tris-HCl pH 7.3, 50 mM NaCl, 50 mM ammonium sulfate, 2 mM ethylenediaminetetraacetic acid (EDTA) and 1 mM dithioerythritol (DTE).

Next, a Heparin XK (GE-Healthcare) column (cv - 30 mL) was equilibrated with 50 mM Tris-HCl pH 7.3, 100 mM NaCl, and 1 mM DTE (binding buffer). Prior to loading the dialyzed protein sample (10 - 20 mL) was diluted to -45 mL with binding buffer and loaded on the column using a 50 mL Superloop (GE-Healthcare). PezTΔ$C_{242}$ was eluted in a step gradient of 60% of buffer B (50 mM Tris-HCl, pH 7.3, 1 M NaCl and 1 mM DTE). Fractions (15 x 1 mL) were collected and analysed by SDS-electrophoresis. PezTΔ$C_{242}$ was concentrated to 100 μM - 200 μM with an Amicon-Ultra 10'000 and injected (1 mL) on a Superdex 75 size exclusion column (GE-Healthcare) equilibrated with 50 mM HEPES-NaOH pH 7.5 and 200 mM NaCl. Fractions (10 x 0.5 mL) were analysed by SDS-electrophoresis and pooled. The protein was concentrated to 200 μM - 300 μM as described above and aliquots shock-frozen using liquid nitrogen. The protein samples were stored at -80°C until use.

**Example 2:** PezT and Zeta toxins phosphorylate UDP-N-acetylglucosamine.

Given the structural similarity of PezT and zeta toxins with phosphotransferases we speculated that they modify UNAG by phosphorylation. Indeed, when we supplied the PezT toxin with UNAG and an excess of ATP and $Mg^{2+}$, we

observed modification of UNAG and turnover of ATP to ADP after separation by anion exchange chromatography (Fig. 1 a).

*PezT/zeta UNAG phosphorylation assay*

Kinase activity of PezTΔ$C_{242}$ and its inhibition by PezA was investigated by incubating 1 μM recombinant toxin in buffer R (25 mM HEPES-NaOH pH 7.5, 100 mM NaCl, and 5 mM $MgCl_2$) supplemented with 1 mM ATP and 0.25 mM of the nucleotide sugars UNAG, UDP-N acetylgalactosamine or UDP-glucose at 25°C for the times mentioned in the respective figure captions (Fig. 1a,b). All nucleotide sugar species were purchased from Sigma. After incubation, samples were diluted 1:2 with $H_20$, filtered and applied to a 1 mL MonoQ column (GE-Healthcare) equilibrated in 50 mM Tris-HCl pH 8.0 at 8°C. The different nucleotide/nucleotide sugar species were eluted with a gradient to 1 M NaCl. Inactivity of the non-toxic variant PezTΔ$C_{242}$ (D66T) was confirmed by performing the same assay using 3 μM of protein. Since inhibition of the S. *pyogenes* zeta toxin by the epsilon antitoxin is much weaker when compared to the PezAT system found in S. *pneumoniae,* we could verify the phosphoryltransfer reaction of the zeta toxin directly by extended incubation (24 h) of 1 μM purified complex with the reaction mixture described above.

In addition to ADP formation, we observed a product that was more negatively charged than the substrate UNAG eluting close to the remaining ATP. This strongly suggested that the PezT toxin phosphorylates UNAG. We could exclude that the observed UNAG modification was the result of a contaminating activity, since addition of stoichiometric amounts of the cognate antitoxin PezA completely inhibited turnover of both UNAG and ATP (Fig. 1 a). Further, we showed that the PezT activity was specific for the presence of the 2' N-acetyl group on the sugar moiety of UNAG since UDP-glucose could not be utilized by PezT. PezT exhibited further selectivity for UNAG as turnover of the stereoisomer UDP-N-acetylgalactosamine was dramatically reduced. Additionally, we showed that the zeta toxin from *S. pyogenes* can catalyze the same reaction as PezT and modifies UNAG using ATP (Fig. 1b). We are therefore of the opinion that the enzymatic function described is a conserved activity of the entire family of zeta toxins. We next investigated whether indeed the PezT toxin phosphorylates UNAG. Thus, we performed electrospray ionization spectrometry experiments, which revealed that the product of the PezT toxin and UNAG differ by the mass of a phosphoryl group ($\Delta m/z = 80$) Using fragmentation by tandem mass spectrometry we found that the phosphorylated UNAG molecule was cleaved into two main fragments, one with a mass corresponding to UDP and one corresponding to N-acetylglucosamine with a phosphoryl group attached to a hydroxyl group. Eventually, we showed by NMR that the PezT toxin attached a phosphoryl group to the 3' hydroxyl group of the N-acetylglucosamine moiety. In conclusion, we have demonstrated that PezT and zeta toxins formed UDP-N-acetyl-3'-phosphate-glucosamine (UNAG-3P) from UNAG using ATP and $Mg^{2+}$ as a cofactor (Fig. 1c).

Ultimately, we could show that the formation of UNAG-3P by PezT is required for cell death *in vivo.* In fact, we were able to identify highly enriched UNAG-3P in low molecular weight-metabolite pools of dying cells obtained by aqueous acetonitrile extraction using high pressure liquid chromatography (Fig. 2a). We confirmed the presence of UNAG-3P in these fractions by chromatography of authentic standards and by mass spectrometry. Cells expressing the enzymatically inactive variant PezT (D66T) did neither die nor accumulate UNAG-3P (Fig. 2a,b).

**Example 3:** MurA activity/inhibition assay

Modification of the aminosugar 3' hydroxyl group by PezT and zeta toxins revealed several possible scenarios in which UNAG-3P could interfere with peptidoglycan synthesis. One of these is inhibition of the conserved enolpyruvyl transferase MurA that catalyzes the initial step of muramic acid synthesis. Subsequent to the hexosamine biosynthesis pathway, MurA modifies the aminosugar 3' hydroxyl group of UNAG (Fig. 3a) and thereby provides the starting point for peptidoglycan biosynthesis. We asked whether zeta toxins simply introduce a branch point in the UNAG metabolism and produce a stable dead-end metabolite or whether the product UNAG-3P is an inhibitor of MurA.

The enolpyruvyl activity of E. *coli* MurA was monitored by coupling phosphate release to cleavage of fluorescent 7-methylguanosine (Sigma) by bacterial purine-nucleoside phosphorylase (PNPase) (Sigma). Each reaction mixture of 400 μL contained 0.3 U PNPase, 50 μM 7-methylguanosine and increasing concentrations of UNAG (0 - 200 μM) in buffer R2 (50 mM NaCl, 50 mM HEPES-NaOH pH 7.5, 1 mM phosphoenolpyruvate) in absence or presence of 15 μM and 30 μM UNAG-3P. UNAG turnover was started by addition of MurA to 0.5 μM. The reactions were monitored by the decrease in fluorescence at 400 nm with excitation set to 300 nm in a FluoroMax-3 spectrofluorometer (HORIBA Jobin Yvon) with excitation and emission bandwidths of 5 nm. After baseline correction, all initial velocities were plotted against the UNAG concentration and fitted globally assuming competitive inhibition of MurA by UNAG-3P using the equation, where $V_{max}$ is the maximal rate in fluorescence decrease at the given MurA concentration, Km the Michaelis-Menten constant of UNAG for E. *coli* MurA and $K_i$ the inhibition constant of UNAG-3P. While the $K_i$ for UNAG-3P converged to 7 μM, the best fit parameter for $K_m$ was 15 μM, which is the same as reported in literature.

$$v = \frac{V_{max} \cdot [UNAG]}{K_m \cdot \left(1 + \frac{[UNAG-3P]}{K_I}\right) + [UNAG]}$$

In fact, increasing UNAG-3P concentrations led to an increase of the apparent $K_m$ of MurA for UNAG, without affecting maximal reaction rate $V_{max}$ in a MurA activity assay (see Fig. 3b). The Michaelis-Menten dataset could be fitted globally by a competitive inhibition mechanism that yielded a $K_i$ of 7 $\mu$M for UNAG-3P in the presence of physiological concentrations of phosphenolpyruvate. We therefore suggest that the phosphate group of UNAG-3P mimics the tetrahedral intermediate during MurA catalysis and is a reversible inhibitor that eliminates turn-over of phosphenolpyruvate due to the blockage of the 3' hydroxyl group of the N-acetylglucosamine moiety (Fig. 3a). Thus, zeta toxins produce a competitive inhibitor for peptidoglycan synthesis.

**Example 4:** Preparative-scale production of UNAG-3P

For preparative-scale production of highly pure UNAG-3P on a milligram basis we established a protocol that can be further up-scaled without difficulty. The yield of this protocol usually exceeds 70 %.

To produce 8 - 10 mg UNAG-3P, 2 x 10 mL of reaction mixture containing 1.6 $\mu$M purified PezT$\Delta C_{242}$, 1.25 mM UDP-N-acetylglucosamine, 1 mM ATP and 5 mM $MgCl_2$ in 200 mM NaCl, 50 mM HEPES-NaOH pH 7.5 were incubated for 5 h at 28°C. Subsequently, each individual 10 mL sample was diluted with 35 mL $H_2O$ and loaded on a MonoQ 10/100 colum (GE-Healthcare, cv = 8 mL) equilibrated with deionized $H_2O$, using a 50 mL superloop. Unreacted UNAG was washed from the column with 3 cv 20% elution buffer (1 M ammonium acetate (volatile) adjusted to pH 8.0 with ammonium hydroxide). UNAG-3P was separated from ADP and residual ATP using a 20 cv gradient from 20% to 100% elution buffer (Fig 4a). Fractions corresponding to UNAG-3P were collected and concentrated using a vacuum concentrator. UNAG-3P was resuspended in 4 mL deionized $H_2O$ and residual salt was removed by injecting the 0.5 mL fractions on a Superdex 75 column equilibrated with deionized $H_20$ (Fig 4b). Fractions containing UNAG-3P were concentrated to 10 - 15 mM as described above and stored at -20°C. The identity UNAG-3P was verified by electrospray ionization mass spectrometry and the spectral purity and concentration was determined by UV absorption spectroscopy ($A_{260}/A_{280}$ = 2.8; $\varepsilon_{262nm}$ = 10100 $M^{-1}$ $cm^{-1}$).

## Claims

**1.** Compound having the general formula (A):

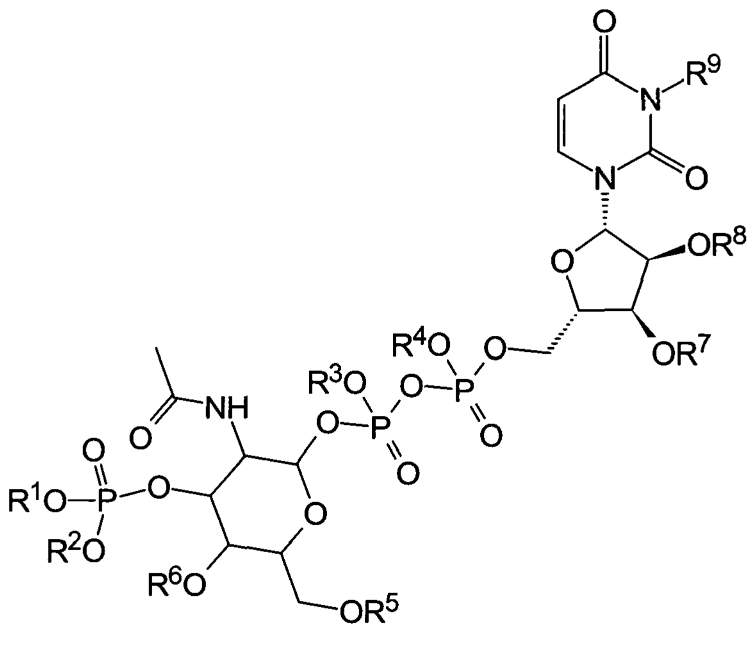

**(A)**

wherein
$R^1$, $R^2$, $R^3$, $R^4$ and $R^9$ represent independently of each other
-H, $-CH_3$, $-C_2H_5$, $-C_3H_7$, $-CH(CH_3)_2$, $-C(CH_3)_3$ $-C_4H_9$, $-C_5H_{11}$, $-C_6H_{13}$, cyclo-$C_3H_5$, cydo-$C_5H_7$, cyclo-$C_5H_9$, cyclo-$C_6H_{11}$, $-CH_2O-COCH_3$, $-CH_{20}-COC_2H_5$, $-CH_2O-COC_3H_7$, $-CH_2O-COC_4H_9$, $-CH_2O-COC_5H_{11}$, $-CH_2O-COC_6H_{13}$, $-CH_2O-COC(CH_3)_3$, $-CH_2O-COOCH_3$ $-CH_2O-COOC_2H_5$, $-CH_2O-COOC_3H_7$ $-CH_2O-COOC_4H_9$, $-CH_2O-COOC_5H_{11}$, $-CH_2O-COOC_6H_{13}$, $-CH_2O-COOC(CH_3)_3$ and $-CH(R^a)-C_6H_4-COOR^b$
wherein $-C_6H_4-$ is a phenylen moiety which is either substituted ortho, meta or para and $R^a$ represents -H or $-CH_2-CO-$

$OR^b$

and $R^b$ represents independently of $R^a$ -H, $-CH_3$, $-C_2H_5$, $-C_3H_7$, $-CH_2(CH_3)$, $-CH(CH_3)_2$, $-C(CH_3)_3$, $-C_4H_9$, $-C_5H_{11}$ or $-C_6H_{13}$;

$R^1$ and $R^2$ can form together one of the following residues

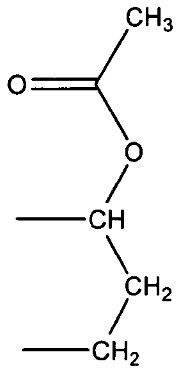
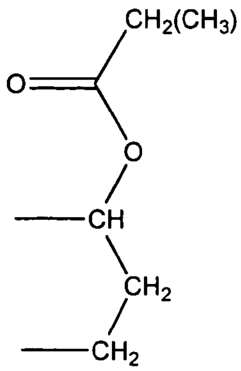
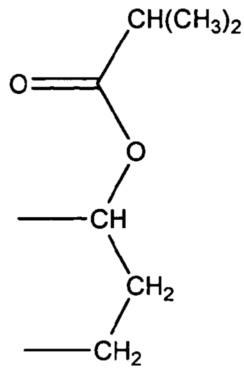
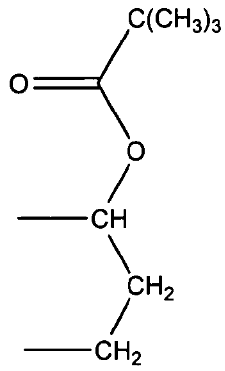

$R^5$, $R^6$, $R^7$, $R^8$ and $R^9$ represent independently of each other -H, -CHO, $-COCH_3$, $-COC_2H_5$, $-COC_3H_7$, $-COCH(CH_3)_2$, $-COC_4H_9$, $-COC_5H_{11}$, $-COC_6H_{13}$, -CO-cyclo-$C_3H_5$, -CO-cyclo-$C_4H_7$, -CO-cyclo-$C_5H_9$, -CO-cyclo-$C_6H_{11}$;

$R^5$ and $R^6$ can form together one of the following residues

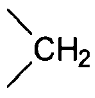
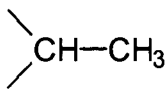
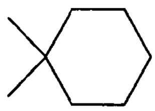
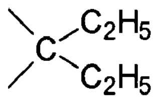
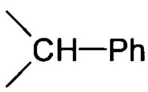
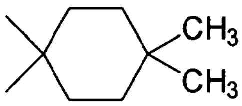
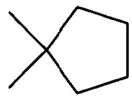
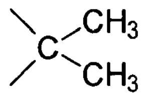
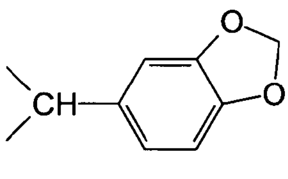
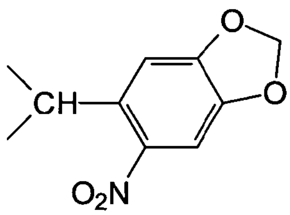
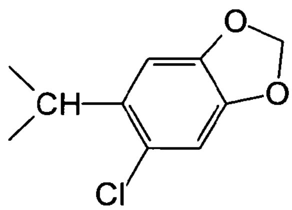

$R^7$ and $R^8$ can form together one of the following residues

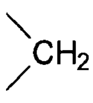
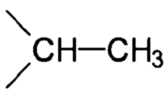
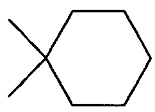
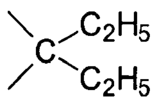
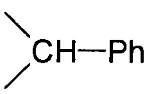
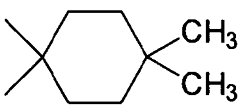
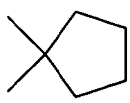
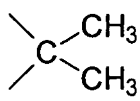

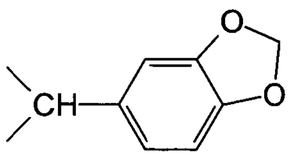 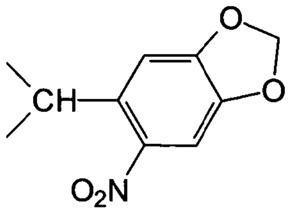 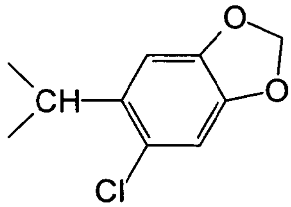

**2.** Compound of claim 1 for use in medicine.

**3.** Use of a compound according to claim 1 for the manufacture of a pharmaceutical composition for treatment of an infectious disease.

**3.** Use of a compound according to claim 1 for treatment of an infectious disease.

**4.** Use according to claim 3, wherein the infectious disease is a bacterial infection.

**5.** Use according to claim 4, wherein the bacterial infection is caused by a bacterium selected from the group comprising:

*Allochromatium vinosum, Acinetobacter baumanii, Bacillus anthracis, Campylobacter jejuni, Clostridium* spp., *Citrobacter* spp., *Escherichia coli, Enterobacter* spp., *Enterococcus faecalis., Enterococcus faecium, Francisella tularensis, Haemophilus influenzae, Helicobacter pylori, Klebsiella* spp., *Listeria monocytogenes, Moraxella catharralis, Mycobacterium tuberculosis, Neisseria meningitidis, Neisseria gonorrhoeae, Proteus mirabilis, Proteus vulgaris, Pseudomonas aeruginosa, Salmonella* spp., *Serratia* spp., *Shigella* spp., *Stenotrophomonas maltophilia, Staphyloccocus aureus, Staphyloccocus epidermidis, Streptococcus pneunmoniae, Streptococcus pyogenes, Streptococcus agalactiae, Yersina pestis,* and *Yersina enterocolitica.*

**6.** Pharmaceutical composition comprising at least one compound according to claim 1 as an active ingredient, together with at least one pharmaceutically acceptable carrier, excipient and/or diluents.

**7.** Method for the production of compounds as defined by claim 1 comprising the steps:

a) Providing a recombinant protein having a sequence of SEQ ID NO:1 or a homolog thereof having the same catalytic activity and
b) Adding ATP and a nucleotide sugar
c) Purifying the compound from the reaction mixture

**8.** Method according to claim 7 further comprising step d):

d) Chemically modifying the compound of step c).

**9.** Method of claim 7 or 8 wherein said nucleotide sugar of step b) is UDP-N-acetylglucosamine or UDP-N-acetylgalactosamine,

Figure 1

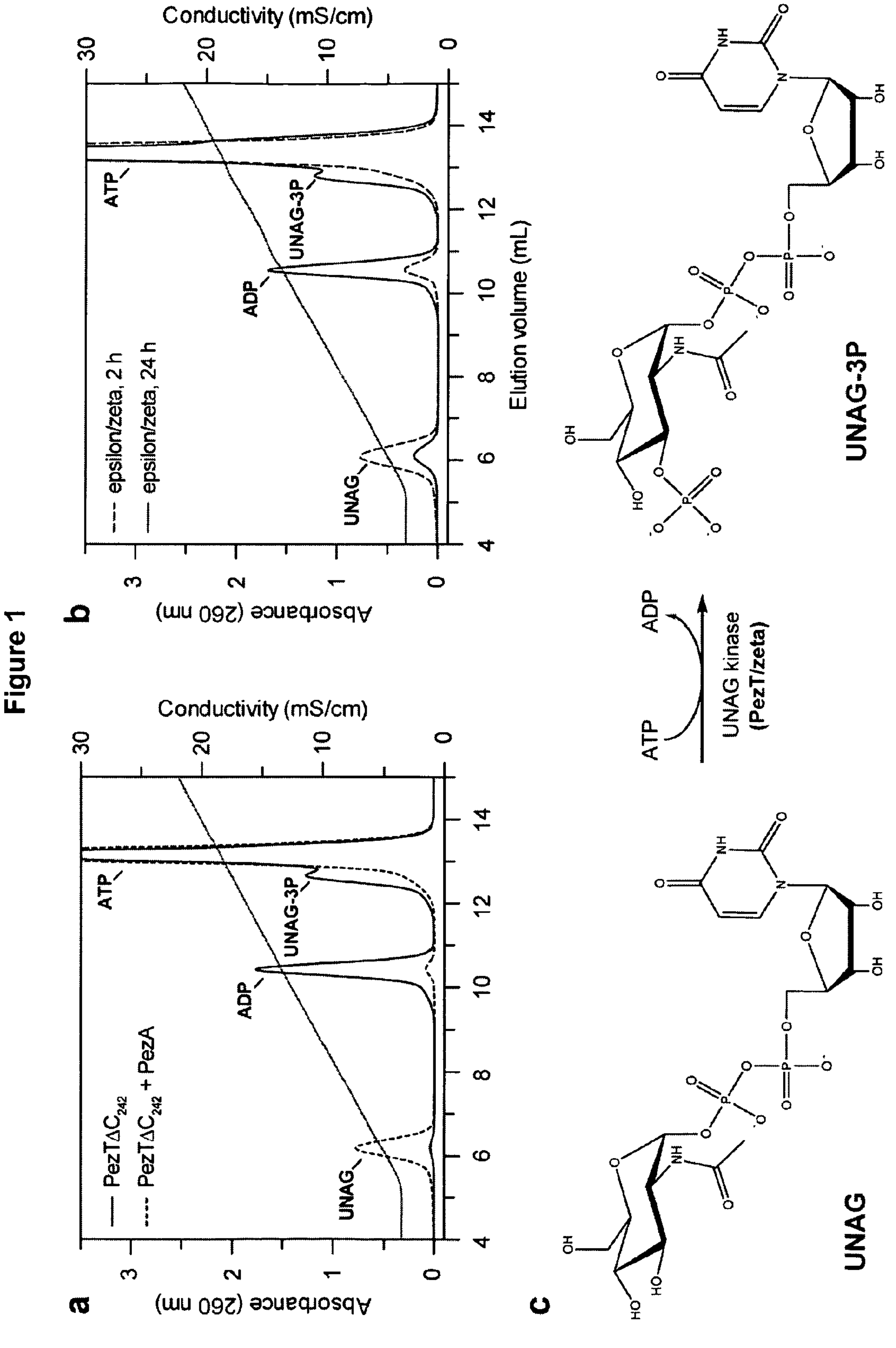


UNAG

UNAG-3P

Figure 2

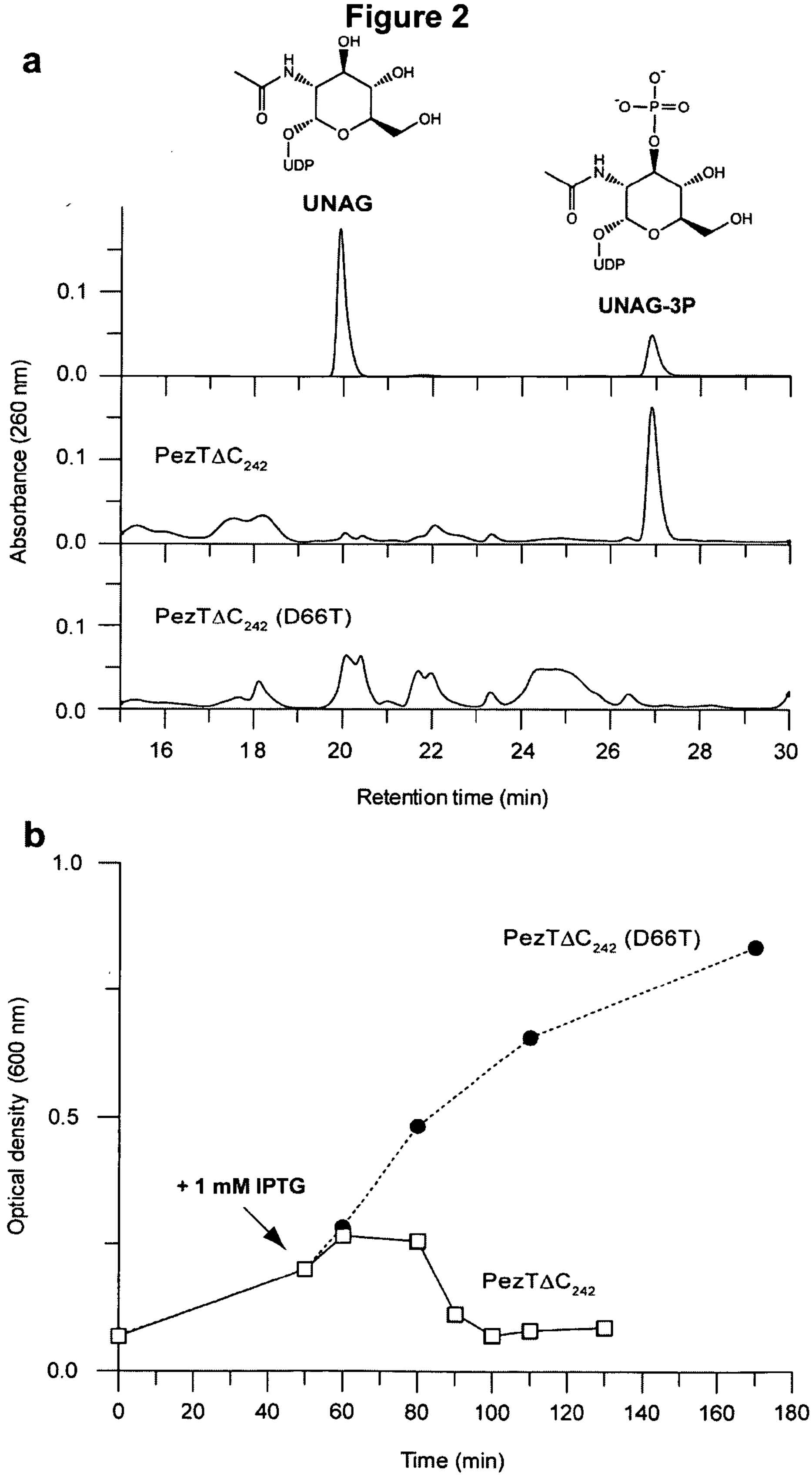
a
UNAG
UNAG-3P
Absorbance (260 nm)
PezTΔC242
PezTΔC242 (D66T)
Retention time (min)
b
Optical density (600 nm)
+ 1 mM IPTG
PezTΔC242 (D66T)
PezTΔC242
Time (min)

# Figure 3

a

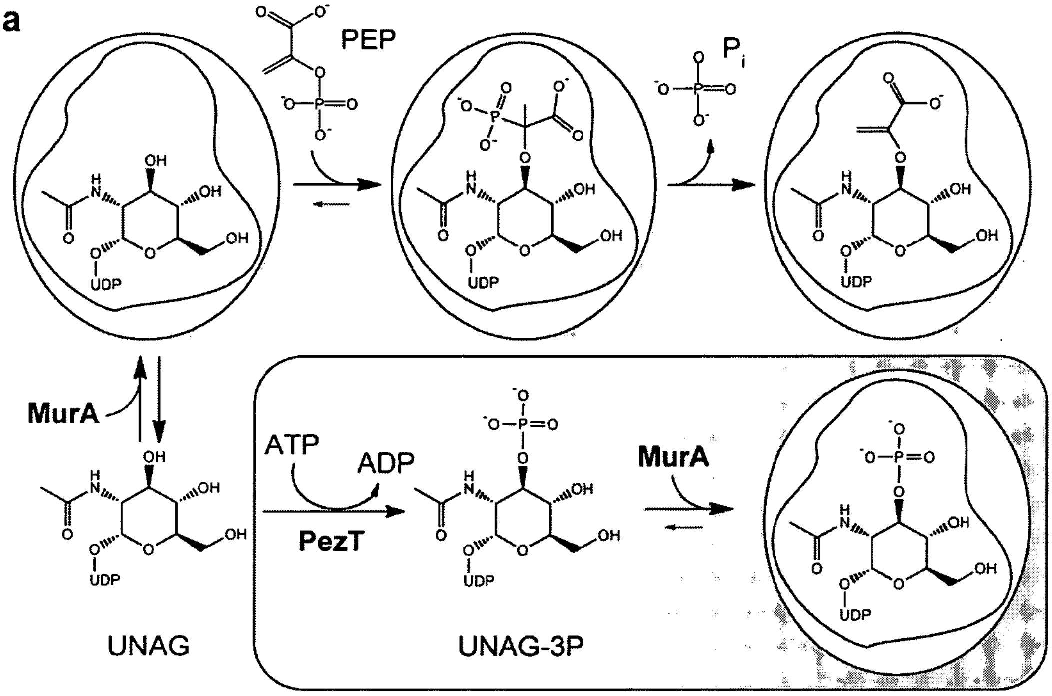


UNAG

UNAG-3P

b

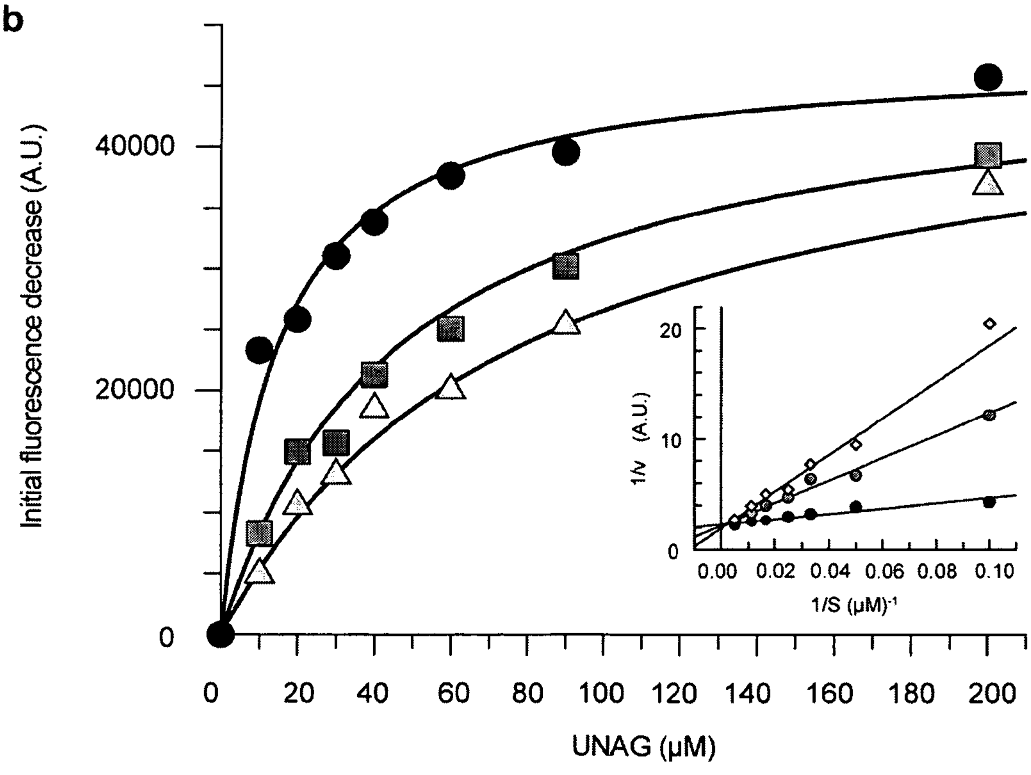

## Figure 4

a

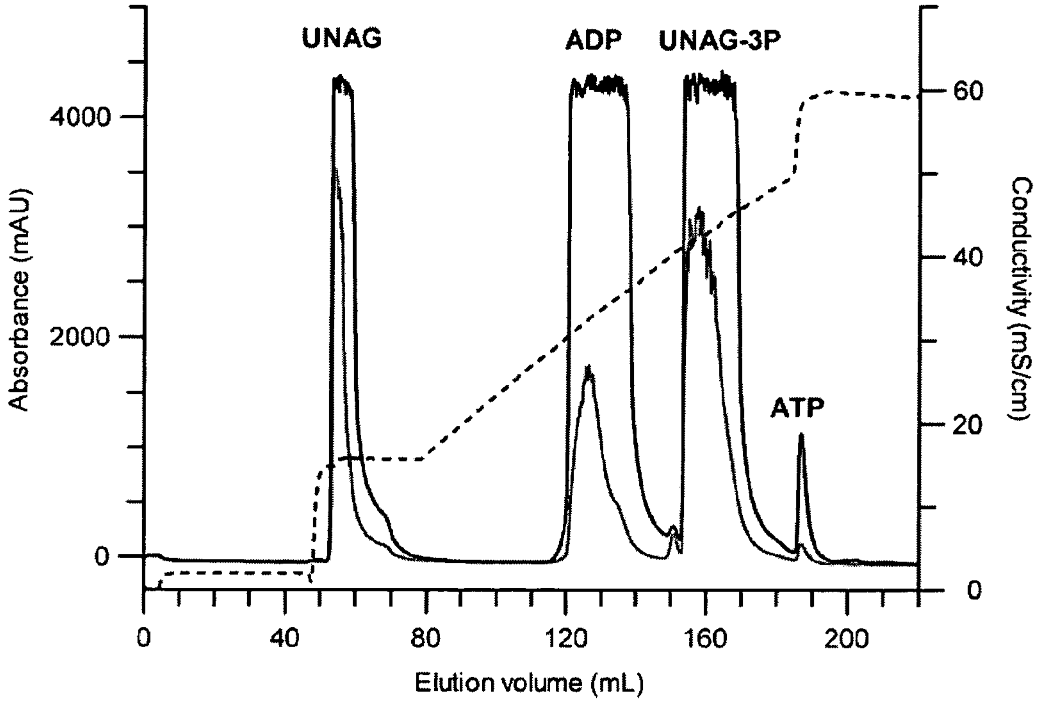
UNAG
ADP
UNAG-3P
ATP
Absorbance (mAU)
Conductivity (mS/cm)
Elution volume (mL)
0
2000
4000
0
20
40
60
0
40
80
120
160
200

b

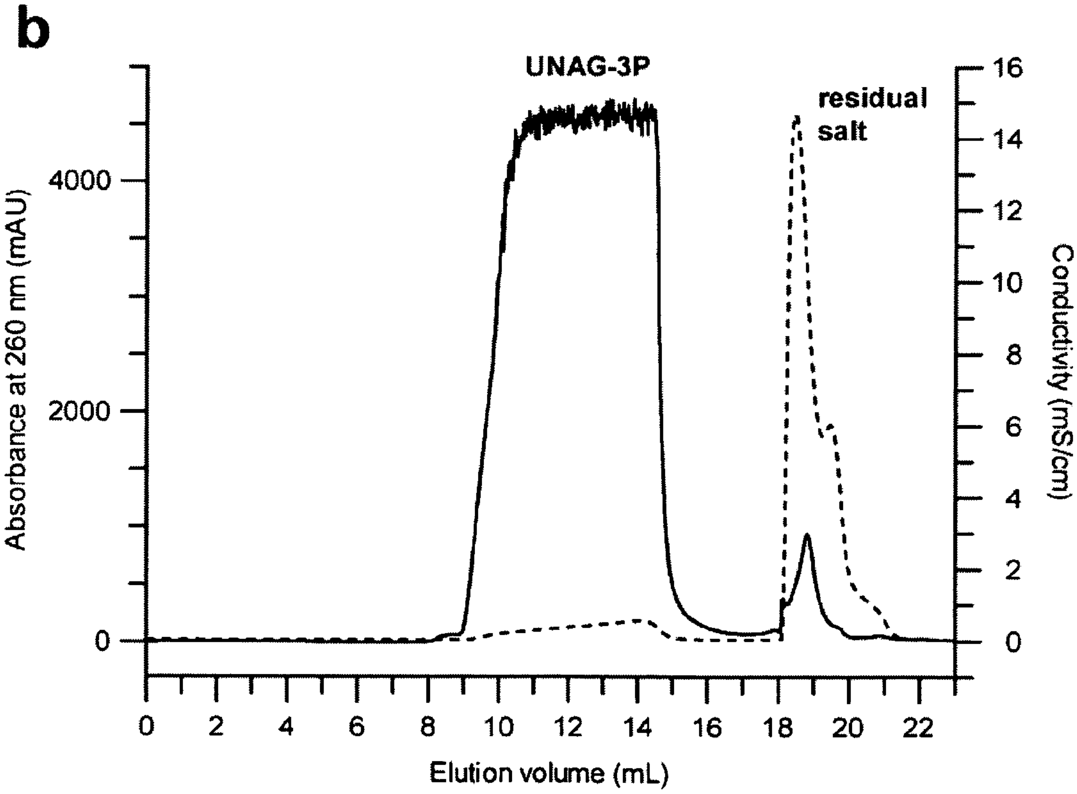
UNAG-3P
residual salt
Absorbance at 260 nm (mAU)
Conductivity (mS/cm)
Elution volume (mL)
0
2000
4000
0
2
4
6
8
10
12
14
16
0
2
4
6
8
10
12
14
16
18
20
22

Europäisches Patentamt
European Patent Office
Office européen des brevets

# EUROPEAN SEARCH REPORT

Application Number
EP 10 07 5522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | S. MIZYED, ET AL.: "UDP-N-acetylmuramic Acid (UDP-MurNAc) is potent inhibitor of MurA (Enolpyruvyl-UDP-GlcNAc Synthase)", BIOCHEMISTRY, vol. 44, 2005, pages 4011-4017, XP002624883, * the whole document * ----- | 1-9 | INV. C07H19/10 A61K31/7072 A61P31/04 |
| A | FOLKERT RECK, ETAL.: "Inhibitors of the Bacterial Cell Wall Biosynthesis Enzyme MurC", BIOORGANIC AND MEDICINAL CHEMISTRY LETTERS, vol. 11, 2001, pages 1451-1454, XP002624884, * the whole document * ----- | 1-9 | |

TECHNICAL FIELDS SEARCHED (IPC)
C07H
A61K

1

The present search report has been drawn up for all claims

| Place of search | Date of completion of the search | Examiner |
|---|---|---|
| Munich | 25 February 2011 | Nikolai, Joachim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WENSENG DU et al.** *J Bacteriol,* 2000
- **H. BARRETEAU et al.** *FEMS Microbiol. Rev,* 2008
- Remington's Pharmaceutical Sciences. Mack Publishing Co,
- **S. K. KHOO et al.** *J. Biol. Chem.,* 2007